# EUROPÄISCHE PATENTANMELDUNG

(11) **EP 0 732 083 A2**
(43) Veröffentlichungstag der Anmeldung: **18.09.1996**
(21) Anmeldenummer: 96103419.6
(22) Anmeldetag: 05.03.1996
(51) Int. Cl.: A61C 19/00, A61C 1/00

(54) **Vorrichtung zum Reinigen wenigstens eines Medienkanals und eines sogenannten Antriebkanals in einem medizinischen, insbesondere zahnärztlichen Handstück**

(30) Priorität: 14.03.1995 DE 19509180
(71) Anmelder: KALTENBACH & VOIGT GmbH & Co., D-88400 Biberach/Riss (DE)
(72) Erfinder: Wiek, Hans-Dieter, 88454 Hochdorf (DE); Eibofner, Eugen, 88400 Biberach (DE)
(74) Vertreter: Schmidt-Evers, Jürgen, Dipl.-Ing.

(57) **Zusammenfassung**

Bei einer Vorrichtung (1) zum Reinigen wenigstens eines Medienkanals (34a, 34b) und eines sogenannten Antriebskanals (35a, 35b) eines medizinischen, insbesondere zahnärztlichen Handstücks (2, 3), mit einem ersten Kupplungsteil (6a, 7a), das mit einem im hinteren Bereich des Handstücks (2, 3) angeordneten zweiten Kupplungsteil (6b, 7b) komplementär ist und das von einer ersten und einer zweiten Zuführungsleitung durchsetzt ist, von denen die erste Zuführungsleitung (32) von einem Anschluß (11) für eine Druckgas-, insbesondere Druckluftquelle, und die zweite Zuführungsleitung (33) von einem Spender (5) für ein vorzugsweise flüssiges Pflege- bzw. Schmiermittel, insbesondere einen Pflegespray, ausgeht, wobei die Zuführungsleitungen (32, 33) im zusammengesteckten Zustand der Kupplungsteile abgedichtet mit wenigstens einem im Handstück (2, 3) weitergeführten Medienkanal (34a, 34b) und Antriebskanal (35a, 35b) verbunden sind, wobei eine Steuereinrichtung (12) mit einem ersten Betätigungsglied (14) vorgesehen ist, durch dessen Betätigung sich der Antriebskanal (35a, 35b) mit dem Spender (5) für das Pflegemittel verbinden läßt, weist die Steuereinrichtung (12) ein zweites Betätigungsglied (13) auf, bei dessen Betätigung der Antriebskanal (35a, 35b) mit dem Druckgasanschluß (11) verbunden ist.

## Beschreibung

Die Erfindung bezieht sich auf eine Vorrichtung nach dem Oberbegriff des Anspruchs 1.

Bei Handstücken mit elektromotorisch oder pneumatisch angetriebenem Werkzeug bedarf es mechanischer Antriebs- und Lagerteile in ihrem Inneren, dem sogenannten Antriebskanal, die aufgrund ihrer Antriebsbewegung einem Verschleiß unterliegen und deshalb gereinigt und geschmiert bzw. gepflegt werden müssen, um eine lange Lebensdauer zu erreichen.

Einer Reinigung in bestimmten Zeitabständen bedarf es auch für den oder die Medienkanäle eines vorliegenden Handstücks. Es ist der Zweck der Medienkanäle, eine Spülflüssigkeit oder Kühlflüssigkeit zur Behandlungsstelle zu führen und dadurch die Behandlungsstelle von Verunreinigungen oder Bearbeitungsspänen freizuspülen und sowohl die Behandlungsstelle als auch das Werkzeug zu kühlen. Insbesondere für den zuletzt genannten Zweck wird bei chirurgischen und insbesondere mikrochirurgischen Handstücken eine Salzlösung als Kühlflüssigkeit verwendet. Die vorbeschriebene Reinigung der Medienkanäle ist erforderlich, weil sich aus den Flüssigkeiten Ablagerungen an den Wandungen der Kanäle anlagern, die zu einer Verengung oder Verstopfung des zugehörigen Kanals führen.

In der DE 31 04 237 C3 ist eine Vorrichtung zur Abgabe von Pflegemittel an ärztliche, insbesondere zahnärztliche Handstücke beschrieben. Diese bekannte Vorrichtung weist einen Aufsteckzapfen als Kupplungsteil auf, das mit einem am hinteren Ende des Handstücks angeordneten Kupplungsteil komplementär ist, so daß das Handstück auf den Aufsteckzapfen aufsteckbar ist. Die Vorrichtung weist einen Schmiermittelvorrat unter Druck in einer Druckflasche aufweist, von der sich eine Schmiermittelzuführungsleitung die Kupplungsteile im gekuppelten Zustand dicht durchsetzend bis in den Antriebskanal des Handstücks erstreckt. Im Aufsteckzapfen ist eine Antriebswelle drehbar gelagert und durch einen Kleinen Elektromotor antreibbar, die im aufgesteckten Zustand des Handstücks mit dessen Antriebswelle in Verbindung steht. Zum Pflegen bzw. Schmieren der Antriebselemente des Handstücks ist im aufgesteckten Zustand ein im Kopfbereich der Vorrichtung angeordnetes Betätigungsglied manuell zu betätigen. Hierdurch wird ein Ventil in der Zuführungsleitung geöffnet und gleichzeitig der Elektromotor eingeschaltet, so daß Schmiermittel in den Antriebskanal des zu behandelnden Handstücks durch den vorhandenen Druck im Vorratsbehälter während der Rotation der Antriebswelle im Handstück geführt wird. Für eine Vorreinigung des Antriebskanals und/oder eine Reinigung der Medienkanäle des Handstücks ist diese bekannte Vorrichtung nicht eingerichtet.

In der WO 88/02621 ist ein Verfahren und eine Vorrichtung zum Freihalten von fluidführenden Leitungen zahnärztlicher Handstücke beschrieben. Diese bekannte Vorrichtung weist ebenfalls einen Aufsteckzapfen zum Aufstecken des zu behandelnden Handstücks auf, wobei zwei die zugehörigen Kupplungsteile durchsetzende Zuführungsleitungen vorgesehen sind, von denen die eine Zuführungsleitung von einer Luftdruckquelle und die andere Zuführungsleitung von einem Schmiermittelbehälter ausgeht, der unter Eigendruck stehen kann oder mit dem Druck der Druckluftquelle beaufschlagbar ist. Diese bekannte Vorrichtung ermöglicht es durch eine entsprechende Betätigung eines Betätigungselements, den vorhandenen Medienkanal im Handstück auszublasen und den Antriebskanal zu schmieren.

Eine Vorrichtung der eingangs angegebenen Art ist in dem Prospekt "KaVo Sprayrotor Clinic 2109" der Anmelderin beschrieben. Diese bekannte Vorrichtung besteht aus einem Ölvorratsbehälter und einem darauf befestigbaren Vorrichtungskopf, an dem zwei Aufsteckzapfen als erste Kupplungsteile einer Steckkupplung für jeweils ein zugeordnetes Handstück angeordnet sind. Die Aufsteckzapfen sind prinzipiell gleich ausgebildet, jedoch ist der eine für ein Handstück mit einem elektromotorischen Antrieb und der andere für ein Handstück mit einem Turbinenantrieb (Druckluft) eingerichtet. In dem einen Aufsteckzapfen ist eine Antriebswelle drehbar gelagert, die durch einen kleinen, im Vorrichtungskopf angeordneten Elektromotor antreibbar ist. Zur Speisung des Elektromotors weist der Vorrichtungskopf einen elektrischen Anschluß auf. Des weiteren weist er einen Druckluftanschluß auf, mit dem er an eine Druckluftquelle anschließbar ist. Die zugehörige Druckluftleitung erstreckt sich sowohl zum Ölvorratsbehälter als auch über eine Ventilanordnung zu beiden Aufsteckzapfen, von denen sie bei aufgesteckten Handstücken sich in Medienkanälen der Handstücke fortsetzt. Mit dieser bekannten Vorrichtung ist es möglich, sowohl die Medienkanäle durch Ausblasen zu reinigen als auch die Antriebskanäle zu schmieren und danach ebenfalls auszublasen und somit nachzureinigen. Hierfür ist an der Oberseite des Vorrichtungskopfes ein Druckknopf vorgesehen, bei dessen manueller Betätigung Öl in den oder die Antriebskanäle und gleichzeitig Luft in die Medienkanäle geblasen wird.

Nach einer bestimmten kurzen Zeit, wenn eine dosierte Ölmenge in den Antriebskanal geblasen worden ist, wird Luft in den Antriebskanal geblasen, um überschüssiges Öl aus letzterem herauszublasen. Der Vorgang läuft ab, solange der Druckknopf gedrückt ist.

Diese bekannte Vorrichtung hat sich in der Praxis als vorteilhaft erwiesen. Sie ist allerdings sehr aufwendig. Außerdem hat es sich herausgestellt, daß das Einspritzen von Öl in den Antriebskanal deshalb nachteilig ist, weil das Öl aufgrund eines Fließverhaltens ein verhältnismäßig großes Verharrungsvermögen besitzt und deshalb der Schmierungseffekt beeinträchtigt ist.

Der Erfindung liegt die Aufgabe zugrunde, bei einer Vorrichtung der eingangs angegebenen Arten die Reinigung und/oder Pflege zu verbessern.

Diese Aufgabe wird durch die Merkmale des Anspruchs 1 gelöst.

Bei der erfindungsgemäßen Vorrichtung ist ein zweites Betätigungsglied vorgesehen ist, bei dessen Betätigung die Schmierung bzw. Pflege des Antriebskanals unabhängig von der Reinigung des oder der Medienkanäle durchgeführt werden kann. Hierdurch kann die Schmierung des oder der Antriebskanäle unabhängig von der Reinigung der Medienkanäle und somit gezielter durchgeführt werden. Außerdem kann die Bedienungsperson die Abschmierzeit selber bestimmen. Es ist somit möglich, daß ein Handstück, das bereits eine lange Zeit nicht geschmiert worden ist, länger dem Schmiervorgang zu unterziehen und ein Handstück, das oft geschmiert worden ist, nur einem kurzen Schmiervorgang zu unterziehen. Hierdurch ist einer individuellen Behandlungsweise und Pflege Rechnung getragen. Außerdem ist es möglich, den oder die Antriebskanäle nach dem Abschmieren von überschüssigem Öl durch Ausblasen zu reinigen und zwar unabhängig vom Schmiervorgang. Bei diesem Reinigungsvorgang kann die Bedienungsperson ein individuelles Erfordernis berücksichtigen und den Reinigungsvorgang kurz oder lang gestalten. Dabei kann die Bedienungsperson diesen Ausblasvorgang unterbrechen und durch Einschalten wieder in Gang setzen. Bei der bekannten Ausgestaltung ist dagegen nach einer Unterbrechung ein nur Ausblasen des Antriebskanals nicht möglich, sondern es erfolgt zunächst wieder ein Abschmiervorgang und dann wieder der Ausblasvorgang.

Dabei ist es im weiteren vorteilhaft, die Anordnung so zu treffen, daß nur ein gemeinsames Betätigungsglied zum Ausblasen des Antriebskanals und des oder der Medienkanäle vorgesehen ist. Hierdurch ist das Reinigen durch Ausblasen der Antriebs- und Medienkanäle zwar kombiniert, jedoch ist dies unschädlich, da ein längeres Ausblasen der Medienkanäle von Vorteil ist, um Ablagerungen von Medienkanalwandungen abzulösen.

In den Unteransprüchen sind Merkmale enthalten, die die mechanische und pneumatische Steuerung in der Vorrichtung verbessern, zu einfachen, kostengünstig herstellbaren und kleinen sowie auch handhabungsfreundlichen Bauweisen führen und außerdem eine einfache und handhabungsfreundliche Steuerung sowie einen einfachen und raumgünstigen elektrischen und pneumatischen Anschluß der Vorrichtung an die erforderlichen Versorgungsmedien ermöglichen. Ferner wird die Schmierung durch die Verwendung einer Schmiermittel-Druckquelle verbessert, die ein Schmiermittel-Luftgemisch unter Druck liefert, das sich im Antriebskanal auch bei engen Querschnitten besser verteilt, wodurch die Schmierung verbessert wird. Des weiteren ermöglichen Weiterbildungsmerkmale der Erfindung eine einfache Halterung eines Schmiermittelvorratsbehälters, die auch einen schnellen und handhabungsfreundlichen Austausch des Schmiermittelbehälters gewährleistet.

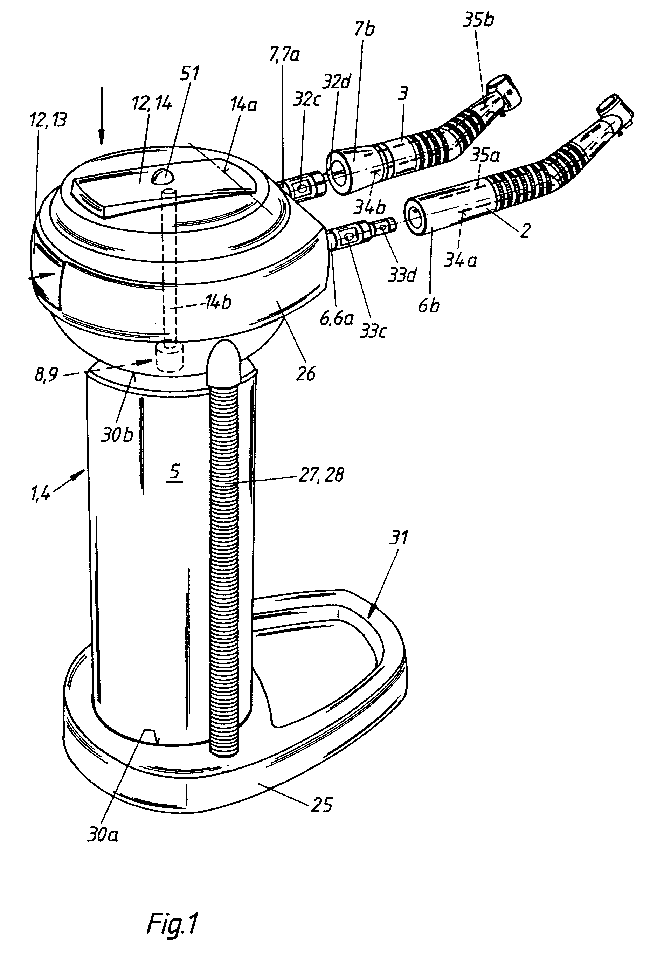

Nachfolgend werden die Erfindung und weitere durch sie erzielbare Vorteile anhand bevorzugter Ausführungsbeispiele und Zeichnungen näher erläutert. Es zeigt
- Fig 1: eine erfindungsgemäße Vorrichtung zum Reinigen wenigstens eines Medienkanals und zum Pflegen eines Antriebskanals in einem medizinischen, insbesondere zahnärztlichem Handstück in perspektivischer Seitenansicht;

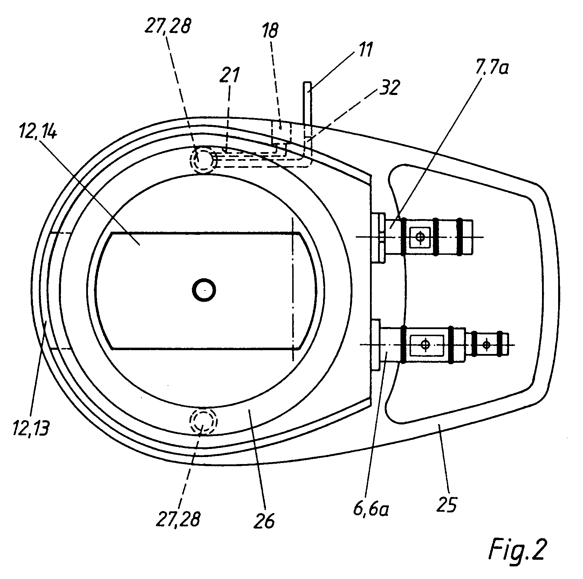

- Fig. 2: die Vorrichtung in der Draufsicht;

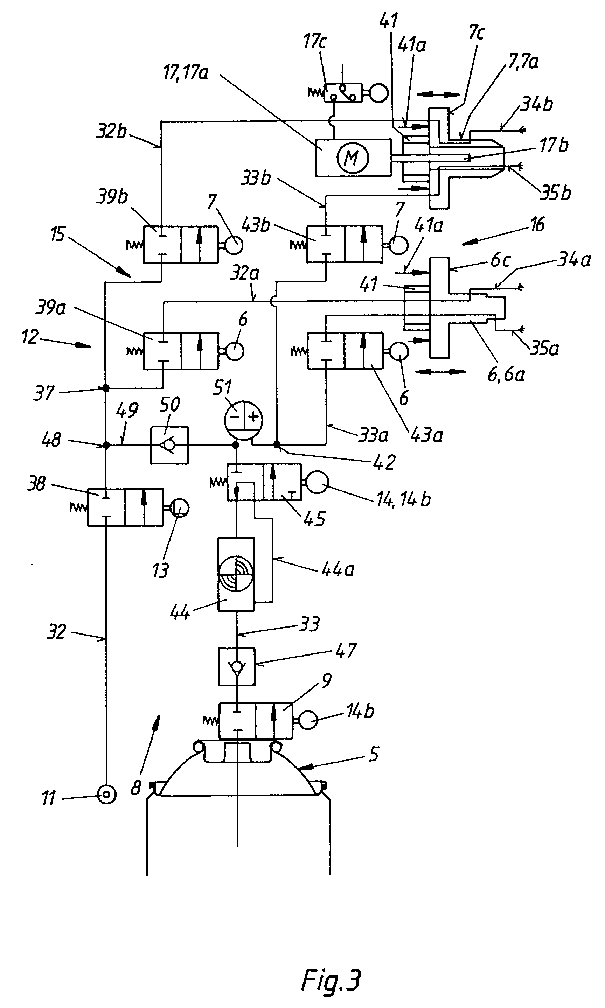

- Fig. 3: einen Steuerplan der Vorrichtung.

Die Hauptteile der allgemein mit 1 bezeichneten Vorrichtung zum Reinigen und Pflegen von andeutungsweise dargestellten, insbesondere zahnärztlichen Handstücken 2, 3 sind ein ständerförmiger Halter 4 für einen ein Pflegemittel unter Druck abgebenden Spender oder Behälter 5, insbesondere eine Spraydose, ein oder mehrere, insbesondere zwei vom Halter 4 vorzugsweise horizontal abstehende Tragzapfen 6, 7, auf die die Handstücke 2, 3 für den Reinigungs- und Pflegevorgang aufsteckbar sind, eine Leitungsanordnung 8 zwischen einem Behälterventil 9 am Kopf des Behälters 5 sowie einem Druckluftanschluß 11 und den Tragzapfen 6, 7, eine Steuerungseinrichtung 12 mit zwei vorzugsweise durch Drucktasten gebildeten Betätigungsgliedern 13, 14, zwei der Leitungsanordnung 8 zugeordneten Ventilanordnungen 15, 16 zur Steuerung der Leitungsverbindung zwischen dem Behälterventil 9 sowie dem Druckluftanschluß 11 und den Tragzapfen 6, 7 und ein dem einen Tragzapfen 7 zugeordneter elektrischer Drehantrieb 17 im Halter 4, der durch ein Leitungskabel 21 mit einem Elektroanschluß 18 verbunden ist. Das Betätigungsglied 13 ist vorzugsweise schubladenförmig verschiebbar gelagert. Das Betätigungsglied 14 ist vorzugsweise um eine horizontale Querachse 14a schwenkbar gelagert.

Der Halter 4 ist durch einen insbesondere plattenförmigen Vorrichtungsfuß 25 und einen darüber in einem Abstand angeordneten Vorrichtungskopf 26 gebildet, zwischen denen der Behälter 5 in aufrechter Position angeordnet und durch eine etwa vertikal wirksame Spannvorrichtung 27 gehalten ist. Die Spannvorrichtung 27 ermöglicht eine vertikale Abstandsveränderung zwischen dem Vorrichtungsfuß 25 und -kopf 26, und sie wird vorzugsweise durch zwei Zugfedern 28, insbesondere Wendelfedern, gebildet, deren Enden zu beiden Seiten des Behälters 5 am Vorrichtungsfuß 25 und - kopf 26 befestigt sind. Hierdurch ist es möglich zwecks Positionierung oder Entfernung oder Austausch des Behälters 5 den Vorrichtungsfuß 25 und -kopf 26 handhabungsfreundlich und schnell auseinanderzuziehen und den Behälter 5 dazwischenzusetzen, wobei eine dichte Leitungsverbindung zwischen dem Behälterventil 9 und der Leitungsanordnung 8 hergestellt wird. Vorzugsweise sind der Vorrichtungsfuß 25 und der -kopf 26 jeweils mit einer oberseitigen bzw. unterseitigen Ausnehmung 30a, 30b an die Form des Behälters 5 angepaßt, so daß letzterer formschlüssig und kraftschlüssig dazwischen gehalten ist. Der Vorrichtungsfuß 25 ist vorzugsweise zur Vorderseite als Ablageteil 31 mit einer Ablagemulde verlängert. Die Tragzapfen 6, 7 stehen vom Vorrichtungskopf 26 zur Vorderseite ab, d.h. über dem Ablageteil 31.

Der Druckluftanschluß 11 und der Elektroanschluß 18 sind vorzugsweise am Vorrichtungsfuß 25, insbesondere seitlich, angeordnet, wobei die zugehörigen Leitungen 21, 32 sich durch eine oder beide Federn 28 zum Vorrichtungskopf 26 hin erstrecken.

Die Tragzapfen 6, 7 bilden jeweils ein erstes Kupplungselement 6a, 7a, das mit einem zweiten Kupplungselement 6b, 7b am zugehörigen Handstück 2, 3 komplementär ist, so daß letztere angekuppelt werden können, hier aufgesteckt werden können. In den Tragzapfen 6, 7 münden jeweils zwei Zuführungsleitungen 32a, 32b, 33a, 33b, an Öffnungen 32c, 32d bzw. 33c, 33d aus, die im Kupplungszustand des zugehörigen Handstücks 2, 3 mit im einzelnen nicht dargestellten Eingangsöffnungen von nur andeutungsweise dargestellten Weiterführungsleitungen 34a, 34b bzw. 35a, 35b durch O-Ringe abgedichtet in Verbindung stehen. Bei den Weiterführungsleitungen 35a, 35b handelt es sich um sogenannte Antriebskanäle, in denen Antriebselemente gelagert sind, wie eine Turbine im Turbinenhandstück 2 und ein Antriebswellenabschnitt im elektrisch betreibbaren Handstück 3. Die Weiterführungsleitungen 34a, 34b dienen im Arbeitsbetrieb des Handstücks 2, 3 als Medienleitung der Zuführung von Luft und/oder Wasser bzw. Spray zur Behandlungsstelle. Die Anordnung der Weiterführungsleitungen ist an sich bekannt und soll hier nicht weiter beschrieben werden.

An der Vorrichtung 1 können wahlweise das Turbinen-Handstück 2 und das elektrisch betreibbare Handstück 3 jeweils einzeln oder gleichzeitig innen gereinigt und gepflegt werden. Bei einer manuellen Betätigung des Betätigungsgliedes 13 durch den Druck von oben werden der oder die Medienkanäle (Weiterführungsleitungen 34a, 35a) und die Antriebskanäle (Weiterführungsleitungen 34b, 35b) durchblasen und gereinigt. Dagegen werden bei einer Betätigung des zweiten Betätigungsglieds 14 durch Druck von der Seite, insbesondere von hinten, die Antriebskanäle (Weiterführungsleitungen 34b, 35b) von dem Pflegemittel durchströmt und somit gepflegt.

Im folgenden werden die Leitungsanordnung 8 und die Ventilanordnungen 15, 16 beschrieben.

Vom Druckluftanschluß 11, der insbesondere seitlich am Vorrichtungsfuß 25 angeordnet ist, erstreckt sich eine Druckluft-Zuführungsleitung 32 durch den Vorrichtungsfuß 25 und durch eine der beiden Zugfedern 28 in den Vorrichtungskopf 26, wo sie am Abzweig 37 in die Zuführungsleitungen 32a, 32b verzweigt, die sich weiter zu den Tragzapfen 6, 7 erstrecken und dort ausmünden wie vorbeschrieben. Stromauf vom Abzweig 37 ist in der Zuführungsleitung 32 ein 2/2-Wegeventil 38 im Vorrichtungskopf 26 angeordnet, das vom ersten, hier seitlichen bzw. rückseitigen Betätigungsglied 13 durch dessen manuelle Betätigung zu öffnen ist und nach dem Loslassen selbsttätig wieder schließt. In den Zuführungsleitungen 32a, 32b ist jeweils ein 2/2-Wegeventil 39a, 39b angeordnet, die jeweils durch das Aufstecken des zugehörigen Handstücks 2, 3 geöffnet werden und nach Entfernung wieder selbsttätig schließen. Dies kann dadurch erfolgen, daß das zugehörige Handstück 2, 3 das zugehörigen Wegeventil 39a, 39b direkt betätigt. Bei der vorliegenden Ausgestaltung sind die Tragzapfen 6, 7 in einer jeweils zugehörigen Führung 41 axial verschiebbar am Vorrichtungskopf 26 gelagert und durch eine Federkraft 41a in ihre ausgeschobene Ausgangsstellung vorgespannt. Durch ein Einschieben des jeweiligen Tragzapfens 6, 7 durch axialen manuellen Druck mit dem aufgesteckten Handstück 2, 3 läßt sich jeweils das zugehörige Wegeventil 39a, 39b öffnen und nach dem Entfernen des Handstücks selbsttätig schließen.

Die Tragzapfen 6, 7 weisen z.B. durch Flansche gebildete Schulterflächen 6c, 7c auf, gegen die die Handstücke 2, 3 zum axialen Verschieben der Tragzapfen 6, 7 drücken.

Die Zuführungsleitungen 33a, 33b zweigen bei 42 von einer gemeinsamen Zuführungsleitung 33 ab, die sich vom Behälterventil 9 im Vorrichtungskopf 26 erstreckt. In den Zuführungsleitungen 33a, 33b ist jeweils ein 2/2-Wegeventil 43a, 43b angeordnet, das in bereits beschriebener Weise beim Aufstecken des zugehörigen Handstücks 2, 3 auf den zugehörigen Tragzapfen 6, 7 geöffnet wird und beim Entfernen des Handstücks 2, 3 selbsttätig wieder schließt. Die Betätigung der Wegeventile 43a, 43b erfolgt auch hier vorzugsweise durch den zugehörigen Tragzapfen 6, 7.

In der Zuführungsleitung 33 ist vorzugsweise stromab eines Dosierventils 44 ein Wegeventil 45 angeordnet, das durch die manuelle Betätigung des Betätigungsglieds 14 geöffnet wird und nach dem Loslassen selbsttätig wieder schließt. Bei der vorliegenden Ausgestaltung handelt es sich um ein 3/2-Wegeventil 45 mit einem Anschluß für eine Dosierleitung 44a des Dosierventils 44. Stromauf des Wegeventils 45 und vorzugsweise auch des Dosierventils 44 ist ein Rückschlagventil 47 in der Zuführungsleitung 33 angeordnet, das in Richtung stromab öffnet.

Zwischen dem Wegeventil 38 und dem Abzweig 37 zweigt an einem und Abzweig 48 eine Verbindungsleitung 49 von der Zuführungsleitung 32 ab, die zwischen dem Wegeventil 45 und dem Abzweig 42 mit der Zuführungsleitung 33 verbunden ist, vorzugsweise stromauf eines Schauglases 51, das an der Oberfläche des Vorrichtungskopfes 26 angeordnet ist, bei der vorliegenden Ausgestaltung an der Oberseite des zweiten Betätigungsgliedes 14.

Der elektrische Drehantrieb 17 umfaßt einen Elektromotor 17a und eine Antriebswelle 17b, die sich in dem als Hülse ausgebildeten Tragzapfen 7 erstreckt, der für das elektrisch antreibbare Handstück 3 bestimmt ist. In der aufgesteckten und einwärts verschobenen Position steht mit der Antriebswelle 17b nicht nur eine weiterführende Antriebswelle im Handstück 3 in Verbindung, sondern es wird - hier ebenfalls durch den verschiebbaren Tragzapfen 7 - ein Schalter 17c betätigt, der den Elektromotor 17a einschaltet und nach dem Loslassen bzw. Entfernen des Handstücks 3 wieder ausschaltet.

Die Funktion der Vorrichtung 1 ist folgende. Zum Pflegen und Reinigen eines der beiden Handstücke 2, 3 oder beider Handstücke 2, 3 zugleich werden diese auf die zugehörigen Tragzapfen 6, 7 aufgesteckt und mit dem oder den Tragzapfen 6, 7 axial gegen den Vorrichtungskopf 26 geschoben, der mit der jeweils anderen Bedienungshand gehalten wird. Dabei werden die zugehörigen Wegeventile 39a, 39b, 43a, 43b geöffnet. Anschließend wird das Betätigungsglied 14 betätigt, über dessen Stössel 14b das Behälterventil 9 geöffnet wird, wobei eine dosierte Menge Pflegespray durch das Schauglas 51, und das zugehörige oder die zugehörigen Wegeventile 43a, 43b sowie den zugehörigen oder die zugehörigen Tragzapfen 6, 7 in den oder die zu pflegenden Antriebskanäle des oder der Handstücke 2, 3 strömt wird und zwar aufgrund des im Behälter 5 enthaltenen Druckes. Sofern ein Handstück 3 oder gleichzeitig ein Handstück 3 aufgesteckt wird, wird durch die axiale Verschiebung und Betätigung des Schalters 17c auch der elektrische Drehantrieb 17 eingeschaltet, wobei der in diesem Handstück 3 enthaltene und beim Aufstecken mit der Antriebswelle 17b gekuppelte Antriebswellenabschnitt gedreht wird, so daß das Pflegespray auch zwischen vorhandene Ritzel und kleine Spalte des Antriebskanals gelangt. Durch ein Sperrventil, insbesondere ein Rückschlagventil 50 in der Verbindungsleitung 49 wird dabei verhindert, daß Pflegemittel in die Zuführungsleitungen 32, 32a, 32b gelangt. Dieser Pflegevorgang dauert solange an, solange das oder die Handstücke 2, 3 bei der vorliegenden Ausgestaltung mit den zugehörigen Tragzapfen 6, 7 gegen die vorhandenen Federn verschoben sind und das Betätigungsglied 14 betätigt bzw. gedrückt ist. Nach dem Loslassen des Betätigungsglieds 14 wird die Pflegemittelzufuhr unterbrochen.

Wenn nach einer solchen Pflege die Medienkanäle und der Antriebskanal bzw. das Handstückinnere zwecks Reinigung mit Druckluft durchblasen werden sollen, ist in der aufgesteckten und weitergeschobenen Schaltstellung des oder der Handstücke 2, 3 und des oder der zugehörigen Tragzapfen 6, 7 das Betätigungsglied 13 zu betätigen, durch dessen Stössel das Wegeventil 38 geöffnet wird und Druckluft durch die Zuführungsleitungen 32a, 32b sowie 33a, 33b zu dem oder den Medienkanälen 34a, 34b und dem oder den Antriebskanälen 35a, 35b gelangt und ausbläst. Dabei werden diese Kanäle gereinigt und gleichzeitig werden der oder die Antriebskanäle von überschüssigem Pflegemittel freigeblasen. Dabei ist auch der elektrische Drehantrieb 17 eingeschaltet, sofern das zugehörige Handstück 3 einzeln oder gleichzeitig behandelt wird. Diese Reinigung durch Ausblasen der Medien- und Antriebskanäle kann wahlweise durch eine Betätigung des Betätigungsgliedes 13 nur oder auch vor dem Pflegen des Antriebskanals erfolgen. Danach können das oder die Handstücke 2, 3 abgezogen und für den Gebrauch bereitgelegt werden.

## Patentansprüche

1. Vorrichtung (1) zum Reinigen wenigstens eines Medienkanals (34a, 34b) und eines sogenannten Antriebskanals (35a, 35b) eines medizinischen, insbesondere zahnärztlichen Handstücks (2, 3), mit einem ersten Kupplungsteil (6a, 7a), das mit einem im hinteren Bereich des Handstücks (2, 3) angeordneten zweiten Kupplungsteil (6b, 7b) komplementär ist und das von einer ersten und einer zweiten Zuführungsleitung (32a, 33a) durchsetzt ist, von denen die erste Zuführungsleitung (32) von einem Anschluß (11) für eine Druckgas- insbesondere Druckluftquelle und die zweite Zuführungsleitung (33) von einem Spender (5) für ein vorzugsweise flüssiges Pflege- bzw. Schmiermittel, insbesondere einen Pflegespray, ausgeht, wobei die Zuführungsleitungen (32a, 33a) im zusammengesteckten Zustand der Kupplungsteile abgedichtet mit wenigstens einem im Handstück (2, 3) weitergeführten Medienkanal (34a, 34b) und Antriebskanal (35a, 35b) verbunden sind, wobei eine Steuereinrichtung (12) mit einem ersten Betätigungsglied (14) vorgesehen ist, durch dessen Betätigung sich der Antriebskanal (35a, 35b) mit dem Spender (5) für das Pflegemittel verbinden läßt,
**dadurch gekennzeichnet,**
daß die Steuereinrichtung (12) ein zweites Betätigungsglied (13) aufweist, bei dessen Betätigung der Antriebskanal (35a, 35b) mit dem Druckgasanschluß (11) verbunden ist.

2. Vorrichtung nach Anspruch 1,
**dadurch gekennzeichnet**,
daß der Spender für das Pflegemittel durch einen unter Druck, insbesondere unter Eigendruck, stehenden Behälter (5), insbesondere eine Spraydose, gebildet ist.

3. Vorrichtung nach Anspruch 1 oder 2,
**dadurch gekennzeichnet**,
daß sie mehrere, insbesondere zwei verschiedene Kupplungsteile (6a, 7a) zum Ankuppeln von verschiedenen Handstücken (2, 3) aufweist.

4. Vorrichtung nach einem der vorherigen Ansprüche,
**dadurch gekennzeichnet**,
daß sie ein auf den Spender (5) aufgesetzbares oder aufgesetztes Kopfteil (26) aufweist und die Betätigungsglieder (13, 14) am Kopfteil (26) angeordnet sind.

5. Vorrichtung nach einem der vorherigen Ansprüche,
**dadurch gekennzeichnet**,
daß das erste Betätigungsglied (14) an der Oberseite des Kopfteils (26) angeordnet ist und vorzugsweise durch eine Drucktaste gebildet ist.

6. Vorrichtung nach einem der vorherigen Ansprüche,
**dadurch gekennzeichnet**,
daß das zweite Betätigungselement (13) seitlich am Kopfteil (26) angeordnet ist, insbesondere an dessen dem Tragzapfen (6, 7) gegenüberliegenden Rückseite und vorzugsweise durch eine Drucktaste gebildet ist.

7. Vorrichtung nach einem der vorherigen Ansprüche,
**dadurch gekennzeichnet**,
daß ein Schauglas (51) für die das Pflegemittel leitende Zuführungsleitung vorgesehen ist, das vorzugsweise an der Oberseite des ersten Betätigungsglieds (14) angeordnet ist.

8. Vorrichtung nach einem der vorherigen Ansprüche,
**dadurch gekennzeichnet**,
daß bei der Betätigung eines Betätigungsgliedes (13) der oder die Medienkanäle (34a, 34b) mit dem Druckgasanschluß (11) verbunden sind.

9. Vorrichtung nach Anspruch 8,
**dadurch gekennzeichnet**,
daß ein gemeinsames zweites Betätigungsglied (13) zum Verbinden des Antriebskanals (35a, 35b) und des oder der Medienkanäle (34a, 34b) mit dem Druckgasanschluß (11) vorgesehen ist.

10. Vorrichtung nach einem der vorherigen Ansprüche,
**dadurch gekennzeichnet**,
daß jeweils ein Ventil (39a, 39b, 43a, 43b) für die erste und die zweite Zuführungsleitung (32, 33) oder Leitungszweige (32a, 32b, 33a, 33b) vorgesehen ist, die beim Kuppeln der Kupplungsteile (6a, 6b; 7a, 7b) geöffnet werden.

11. Vorrichtung nach Anspruch 10,
**dadurch gekennzeichnet**,
daß das oder die ersten Kupplungsteile (6a, 7a) gegen eine Rückstellkraft axial verschiebbar gelagert ist bzw. sind.

12. Vorrichtung nach Anspruch 10 oder 11,
**dadurch gekennzeichnet**,
daß stromauf von den Ventilen (39a, 39b, 43a, 43b) jeweils ein weiteres Ventil (38, 45) in der ersten und zweiten Zuführungsleitung (32, 33) angeordnet sind, die durch die Betätigungsglieder (13, 14) zu öffnen sind, wobei die erste und die zweite Zuführungsleitung jeweils zwischen den zugehörigen Ventilen (38, 39a, 39b; 43q, 43b, 45) durch eine Verbindungsleitung (49) miteinander verbunden sind, die vorzugsweise durch ein besonderes Ventil sperrbar ist.

13. Vorrichtung nach Anspruch 12,
**dadurch gekennzeichnet**,
daß in der Verbindungsleitung (49) ein in Richtung auf die zweite Zuführungsleitung (33) öffnendes Rückschlagventil (50) angeordnet ist.
